# FASCICULE DE BREVET EUROPEEN

(11) **EP 0 342 128 B1**
(45) Date de publication et mention de la délivrance du brevet: **26.04.1995**
(21) Numéro de dépôt: 89401342.4
(22) Date de dépôt: 12.05.1989
(51) Int. Cl.: C07H 21/04, C07K 2/00, A61K 39/42, C12Q 1/70, G01N 33/569

(54) **Sondes à papillomavirus (HPV49, HPV50, HPV54, HPV55), produits génétiquement et immunologiquement liés à ce papillomavirus et procédé de diagnostic in vitro d'infection à papillomavirus et de production d'anticorps contre ces papillomavirus**
Sonden für Papilloma-Virus (HPV49, HPV50, HPV54, HPV55), zu diesem Papilloma-Virus genetisch und immunologisch verwandte Produkte und in vitro Methoden zur Diagnose von Papilloma-Virusinfektionen und Herstellung von Antikörpern gegen diese Papilloma-Viren
Papillomavirus probes (HPV49, HPV50, HPV54, HPV55), products genetically and immunologically related to this papillomavirus and in vitro methods for the diagnosis of papillomavirus infections and for the production of antibodies against these papillomaviruses

(30) Priorité: 13.05.1988 FR 8806486; 21.06.1988 FR 8808324
(43) Date de publication de la demande: 15.11.1989
(73) Titulaire: INSTITUT PASTEUR, 75724 Paris Cédex 15 (FR)
(72) Inventeur: Orth, Gérard, F-92330 Sceaux (FR); Favre, Michel, F-75015 Paris (FR); Kremsdorf, Dina, F-75013 Paris (FR); Pehau-Arnaudet, Gérard, F-93100 Montreuil (FR)
(74) Mandataire: Gutmann, Ernest

(56) Documents cités:
- EP-A- 0 192 001
- EP-A- 0 235 004
- EP-A- 0 243 221
- WO-A-87/01375
- CHEMICAL ABSTRACTS, vol. 108, mai 1988, page 165, résumé no. 162383c, Columbus,Ohio, US; G.J. NUOVO et al.: "Isolation of a novel human papillomavirus (type51) from a cervical condyloma",& J. VIROL. 1988, 62(4), 1452-5-5

## Description

L'invention concerne des ADNs de papillomavirus (HPV49, HPV50, HPV54, HPV55) ou des variants de ces papillomavirus, et plus particulièrement des sondes dérivées de ces papillomavirus (HPV49, HPV50, HPV54, HPV55). Elle concerne aussi des produits génétiquement et immunologiquement liés à ce papillomavirus, ainsi que des procédés mettant en oeuvre ces divers produits pour ie diagnostic in vitro d'infections à papillomavirus et, pour certains d'entre eux, pour la vaccination contre ces mêmes papillomavirus ou variants de papillomavirus.

Par l'expression "produit génétiquement ou immunologiquement liés à un papillomavirus", il faut entendre les divers produits dérivés de son ADN initial, qu'il s'agisse des ARNs correspondants ou d'ADN recombinants contenant tout ou partie de cet ADN initial, ainsi que les produits "immunologiques" résultant des expressions de ces ADNs, le cas échéant recombinants, dans des hôtes cellulaires compétents. Il s'agit donc de polypeptides résultant de la transcription et de la traduction de tout ou partie des différentes phases de lecture ouvertes de l'ADN initial. Il s'agit encore d'anticorps induits in vitro par lesdits polypeptides.

L'expression "papillomavirus" recouvre un grand nombre de virus ayant en commun d'être tenus pour responsables de plusieurs formes d'infections virales s'étageant entre les verrues cutanés ou de muqueuses relativement bénignes et des hyperplasies susceptibles de dégénérer en néoplasies intra-épithéliales et en cancers cutanés. Parmi des infections à papillomavirus, on mentionnera également plus particulièrement les verrues cutanées (en particulier, verrues vulgaires et plantaires), les épidermodysplasies verruciformes, les verrues planes ou intermédiaires cutanées, les néoplasies intraépithéliales et cancers cutanés, les cancers de l'épidermodysplasie verruciforme, les néoplasies génitales et cancers du col de l'utérus, les condylomes et papillomes.

Un certain nombre de types de papillomavirus a déjà été décrit. On mentionnera à titre d'exemples ceux décrits dans le brevet principal français n° 84.18369/2.578.267 et ses certificats d'addition n° 85.07073/2.581.655 et n° 86.01425/2.593.828, tous relatifs à des types et sous-types nouveaux de papillomavirus qui ont été isolés partir de verrues ou lésions maculaires disséminées, parmi lesquels certains davantage susceptibles que d'autres de donner lieu au développement précoce de cancers de la peau chez les malades qui en sont affectés.

On connaît également l'importance de facteurs immunitaires et le rôle majeur de divers types de virus de papillomes humains (HPV), ces facteurs s'ajoutant au rôle déjà décrit dans la littérature de facteurs génétiques divers et des rayonnements actiniques dans la pathogénèse des infections aux papillomavirus.

L'invention du brevet principal et de ses certificats d'addition découlait des observations qui avaient été faites quant aux comportements relatifs d'un grand nombre de papillomavirus nouvellement isolés, dont les caractéristiques génomiques essentielles ont été définies.

Plusieurs types et sous-types de papillomavirus nouveaux ont été décrits dans le brevet principal et les certificats d'addition. Il en a été de même de l'utilisation des ADNs de ces papillomavirus nouveaux, pris isolément ou en association, entre eux et/ou avec des ADNs d'HPV antérieurement connus, dans des techniques de diagnostic in vitro plus affinées.

D'une façon générale, il a été remarqué dans le brevet principal que les papillomavirus, bien que très différents entre eux, avaient des génomes ayant des tailles de l'ordre de 7000-8000 paires de bases. En outre, leurs génomes peuvent néanmoins présenter certains degrés d'homologie, évalués en pourcentages d'homologies, dans des essais d'hybridation réalisés dans des conditions dites "non stringentes" ou "non strictes" ou, au contraire, dans des conditions "d'hybridation stringente" ou "stricte".

Il a été dit que les papillomavirus qui présentent des pourcentages d'homologie inférieurs à 50 % dans des conditions strictes ou stringentes appartenaient à des types différents. Les virus pour lesquels on observe, dans ces conditions strictes ou stringentes, des pourcentages d'homologie supérieurs à 50 % sont considérés comme appartenant au même type. Ils peuvent former des sous-types différents au sein de ce même type.

Les essais d'hybridation dans les conditions non strictes ou non stringentes impliquent la mise en contact mutuelle d'ADNs provenant de deux isolats de virus dans les conditions suivantes décrites par HEILMAN C.A. et al, 1980, J.Virol., 36, 395-407, et CROISSANT et al, 1982, C.R. Acad. Sc. Paris, 294, 581-586 (molécules hétéroduplexes). En particulier les conditions "non stringentes" impliquent la mise en contact de l'ADN du papillomavirus étudié, d'un variant de ce papillomavirus ou d'un clone de cet ADN avec l'ADN du papillomavirus de référence à une température inférieure d'environ 40°C à la température de fusion (Tm) des hybrides.

Les essais d'hybridation dans les conditions strictes ou stringentes impliquent la mise en contact mutuelle d'ADNs provenant de deux isolats de virus dans les conditions décrites par KREMSDORF, D. et al. ((1982), J. Virol. 43:436-447 et 1983, J. Virol. 48:340-351) et Davis R.W. et al., 1971, Methods Enzymol., 21, 413-418 (molécules hétéroduplexes). En particulier les conditions "stringentes" impliquent la mise en contact de l'ADN du papillomavirus étudié, d'un variant de ce papillomavirus ou d'un clone de cet ADN avec l'ADN du papillomavirus de référence à une température inférieure d'environ 20°C à la température de fusion (Tm) des hybrides.

Plusieurs virus ont été décrits dans le brevet principal et les certificats d addition. De même ont été décrits des recombinants génétiques comprenant tout ou partie des génomes (dénommés ADN-HPVs) de ces virus. L'invention du brevet principal et des certificats d'addition concernait par conséquent des ADN-HPVs ayant en commun des tailles s'étageant entre 7000 et 8000 paires de bases et caractérisés par les cartes de restriction apparaissant dans les dessins de ces brevets et certificats d'addition. En particulier les dessins fournissent plus particulièrement les cartes de restriction de ADN-HPVs obtenus à partir des papillomavirus et qui répondent aux désignations HPV2d, HPV10b, HPV14a, HPV14b, HPV15, HPV17a, HPV17b, HPV19, HPV20, HPV21, HPV22, HPV23, HPV24, HPV28, HPV29, HPV32 (anciennement HPV31), HPV33 (anciennement HPV32 ou HPV-IP2), HPV34 (anciennement HPV-IP3), HPV36 (anciennement HPV-IP4), HPV39 (anciennement IP5) et HPV42 (anciennement IP6).

L'invention du brevet principal et de ces certificats d'addition concernait également des mélanges ou "cocktails" d'ADN-HPVs isolés à partir des papillomavirus nouveaux à l'époque et/ou à partir de papillomavirus antérieurement connus, aux dates de dépôt des titres correspondants, ou des sondes d'hybridation les contenant, susceptibles d'être mis en oeuvre plus efficacement pour le diagnostic de diverses catégories d'infections, voire des niveaux de risques qui accompagnent la découverte chez un patient de papillomavirus déterminés. La découverte de nouveaux papillomavirus ou des ADNs qui en sont dérivés fournit donc la possibilité de réaliser des diagnostics plus affinés, notamment une plus grande discrimination des diverses catégories d'infections imputables aux divers types de papillomavirus ou suceptibles de se développer sous l'effet de ces derniers types et, au sein d'une catégorie d'infections déterminées, de mieux pronostiquer le degré de risque que ces dernières se transforment en des maladies plus redoutables.

A ce titre, la présente invention concerne des papillomavirus nouvellement isolés, les ADNs génomiques qui peuvent en être extraits ou des fragments de ces ADNs génomiques, ainsi que de nouvelles sondes d'hybridation qui peuvent être formées à partir de ces ADN-HPV ou fragments d'ADN-HPV. Elle concerne également tous les variants de ces papillomavirus, appartenant respectivement aux mêmes sous-types, donc capables de s'hybrider avec les papillomavirus correspondants identifiés ci-après par référence à leurs cartes de restriction dans des conditions stringentes.

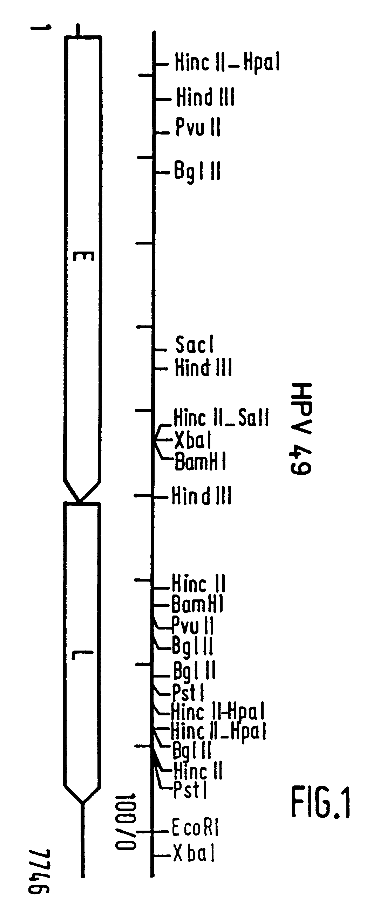

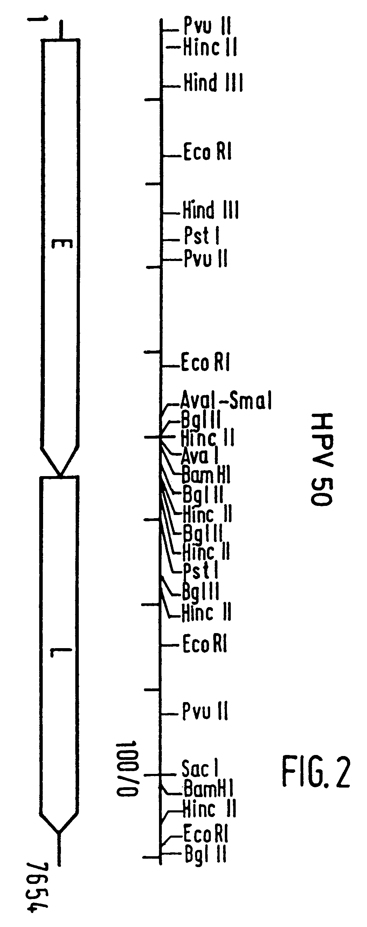

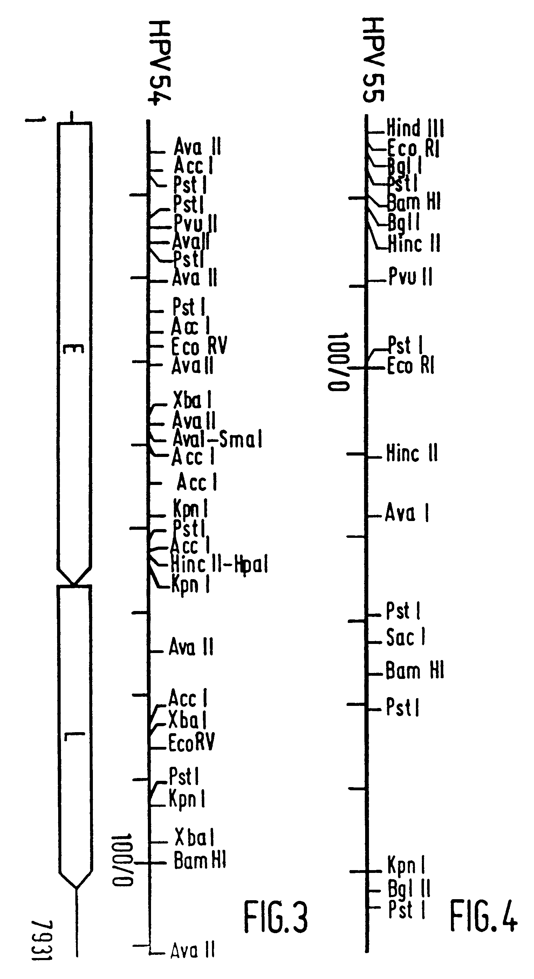

Les ADN-HPV selon la présente invention, ci-après dénommés HPV49 déposé à la CNCM sous le numéro de dépôt I-754, HPV50 déposé à la CNCM sous le numéro de dépôt I-755, HPV54 déposé à la CNCM sous le numéro de dépôt I-756 ou HPV55 notamment HPV55A déposé à la CNCM sous le numéro de dépôt I-757 ou HPV55B déposé à la CNCM sous le numéro de dépôt I-758, sont caractérisés par les cartes de restriction faisant l'objet des figures jointes (figures 1 à 4).

Les cartes physiques donnent la position de sites de coupure par diverses endonucléases de restriction. L'origine de chaque carte est constituée par un site unique de coupure. Les distances des autres sites par rapport à l'origine sont exprimées en pourcentage de longueur de génome.

L'invention concerne encore des "cocktails" nouveaux de sondes comprenant certains des "cocktails" de sondes déjà décrits dans le brevet principal ou les certificats d'addition, cependant complétés avec une sonde dérivée de l'un des papillomavirus selon la présente demande et, par conséquent, des trousses ou "kits" de diagnostic encore davantage perfectionnés.

Les sondes d'hybridations construites à partir de génomes des HPV (HPV49, HPV50, HPV54, HPV55) sont particulièrement utiles pour le diagnostic et/ou le dépistage in vitro, dans les conditions qui ont été décrites dans le brevet principal et les certificats d'addition et qui seront encore décrites plus loin, des types de papillomavirus qui constituent un risque, notamment:
- pour HPV49 : les verrues cutanées (en particulier, verrues vulgaires et plantaires) et le diagnostic différentiel de l'épidermodysplasie verruciforme,
- pour HPV50 : les épidermodysplasies verruciformes, les néoplasies intra-épithéliales et les cancers cutanés,
- pour HPV54 : les néoplasies génitales et les cancers du col de l'utérus,
- pour HPV55 : les néoplasies génitales et cancers du col de l'utérus, les condylomes et papillomes.

Ces sondes d'hybridation sont avantageusement incorporées aux "cocktails" de diagnostic des affections du même type sur lesquels il est revenu plus loin. A cet égard, il sera encore fait référence au brevet principal et aux certificats d'addition dans lesquels figurent les cartes de restriction de certains autres consituants de ces mélanges de diagnostic. A ce titre le brevet principal et les certificats d'addition doivent être considérés comme faisant partie de la présente description, pour ce qui est de l'identification des autres ADN-HPVs entrant dans la composition de ces "cocktails".

L'invention concerne également des fragments de chacun des ADN-HPV précédents ou capables de s'hybrider avec ceux-ci, notamment dans des conditions strictes, de façon à pouvoir détecter l'hybride formé. De même, elle concerne les ADNs recombinants contenant tout ou partie des ADN-HPV sus-indiqués, et plus particulièrement, respectivement, des ADNs recombinants contenant des fragments correspondant aux gènes E1, E2, E6-E7, L1, L2 et à la région intergénique, NC, ou encore des fragments contenant des séquences correspondant aux régions intergéniques de chacun desdits ADN-HPV. Elle concerne aussi les sondes qui peuvent être constituées à partir de ces ADN-HPV ou à partir des fragments correspondants et les procédés de diagnostic in vitro mettant en jeu lesdites sondes ou les mélanges les contenant.

Les préparations des ADNs viraux ont été extraites selon les techniques décrites ci-après.

Dans ce qui suit sont décrites de façon plus précise les conditions dans lesquelles chacun des papillomavirus selon l'invention a été isolé, puis les conditions dans lesquelles l'ADN-HPV a été obtenu à partir de ce papillomavirus.

### Clonage moléculaire et caractérisation d'un nouveau type d'HPV (HPV 49) trouvé dans une verrue d'un patient immunosupprime :

Un nouveau type d'HPV a été mis en évidence dans l'ADN extrait d'une verrue intermédiaire des mains d'un transplanté rénal, par hybridation, dans des conditions non strictes, avec une sonde d'ADN radioactive spécifique du HPV5. Une étude de la sensibilité de l'ADN de cet HPV à des enzymes de restriction a montré que l'endonucléase EcoRI coupe une fois l'ADN viral. Après traitement de l'ADN tumoral par l'endonucléase EcoRI, les produits de digestion ont été insérés par EcoRI dans l'ADN du vecteur plasmidique pGem4. Les plasmides recombinants ayant -intégré l'ADN du nouvel HPV ont été sélectionnés par la technique d'hybridation sur colonies (Benton et Davis), en utilisant une sonde d'ADN radioactive spécifique de HPV5, dans des conditions non strictes. Un plasmide recombinant contenant la totalité des séquences virales a été isolé. La coupure de l'ADN du plasmide recombinant et de la préparation d'ADN extrait de la verrue, par le mélange des endonucléases EcoRI et HindIII, engendre les mêmes quatre fragments d'ADN viral, dont le poids moléculaire correspondant à celui d'un génome de papillomavirus (environ 8 kilobases).

Une carte de restriction de l'ADN du nouvel HPV a été construite à partir de l'étude de la sensibilité de cet ADN à 18 endonucléases de restrictio. Vingt-sept sites de coupure ont été localisés (Annexe 1). Cette carte est différente de celle de tous les HPV isolés à ce jour. Le degré d'homologie entre l'ADN du nouvel HPV et l'ADN des HPV identifiés à ce jour a été analysé par des expériences d'hybridation sur réplique, dans des conditions plus ou moins strictes, en utilisant une sonde d'ADN radioactive spécifique du nouvel HPV. Seule une homologie de séquence a été détectée dans les conditions non strictes d'hybridation entre l'ADN du nouvel HPV et l'ADN des HPV identifiés à ce jour. L'hybridation croisée la plus importante a été observée avec l'ADN des HPV spécifiquement associés à l'épidermodyslasie verruciforme. Le nouveau virus caractérisé à partir d'une verrue d'un patient immunosupprimé constitue donc un nouveau type d'HPV provisoirement dénommé HPV49.

L'analyse, au microscope électronique, de molécules hétéroduplexes formées entre l'ADN du HPV49 et l'ADN du HPV5 a permis de définir la position théorique des différents gènes par l'ADN du HPV49. De même l'établissement de la séquence nucléotidique d'un fragment d'ADN du HPV49 a permis d'aligner la carte physique de l'ADN du HPV49 avec la carte génétique du HPV5.

Le pouvoir pathogène du HPV49 a été recherché à l'aide de sondes radioactives préparées à partir de l'ADN de ce virus. Le HPV49 a été recherché dans des verrues prélevées chez des patients immunosupprimés (27 cas), des bouchers (26 cas) ou dans la population générale (21 cas), dans des lésions bénignes de 31 patients atteints d'EV, dans 18 cas de kératoacanthomes, dans 20 cas de maladie de Bowen cutanée, dans 25 cas de kératose acténique, dans 25 cas d'épithélioma basocellulaire, dans 23 cas d'épithélioma spinocellulaire. Le HPV 49 a été trouvé chez deux autres patients immunosupprimés.

Le HPV49, bien que rare, constitue un type additionnel d'HPV cutané, associé aux verrues. Il convient de l'incorporer à tout mélange d'ADN de HPV destiné à la préparation de sondes moléculaires, en vue du diagnostic des types d'HPV associés aux verrues en particulier chez les sujets immunosupprimés.

Dans le tableau qui suit sont précisées les localisations putatives des principaux gènes et de la région intergénique du HPV49 sur la carte de ce génome :

| | Coordonnées des extrémités (%) | |
|---|---|---|
| | 5′ | 3′ |
| E6-E7 | 2 | 12,5 |
| E1 | 11 | 34,5 |
| E2 | 34 | 54 |
| L2 | 54,5 | 76 |
| L1 | 76 | 97 |
| Région intergénique | 97 | 2 |

### Clonage moléculaire et caractérisation d'un nouveau type d'HPV associé à des lésions de kératose actinique (HPV50):

Un nouveau type d'HPV a été mis en évidence dans l'ADN extrait du mélange de lésions de kératose actinique prélevées chez un malade atteint d'épidermodysplasie verruciforme (EV), par hybridation, dans des conditions non strictes, avec une sonde d'ADN radioactive spécifique de HPV5. Cette préparation d'ADN tumoral avait été préalablement montrée contenir les ADN des HPV5, 20, 23 et 36. Un fragment d'ADN d'environ 1,9 Kb, correspondant à l'ADN du nouvel HPV, a été observé après digestion de l'ADN extrait des lésions par l'endonucléase de restriction EcoRI. L'ADN tumoral a été traité par l'enzyme EcoRI, et les produits de digestion ont été insérés par le site EcoRI dans l'ADN du bactériophage λGt10 permettant le clonage de fragments d'ADN dont la taille est inférieure à 7 kb. Après encapsidation de l'ADN recombinant et infection de bactéries Escherichia Coli K12 (souche C600), les bactériophages recombinants ayant inséré le fragment d'ADN de 1,9 kb ont été sélectionnés par la technique d'hybridation sur plages de lyse, en utilisant une bande d'ADN radioactive spécifique du HPV5 dans des conditions non strictes. Plusieurs bactériophages recombinants ont été isolés et montrés contenir le fragment d'ADN de 1,9 Kb. Ce fragment d'ADN a été excisé des séquences d'ADN phagique et recloné dans le plasmide SP65. Une sonde radioactive préparée à partir du fragment d'ADN de 1,9 Kb, excisé et purifié des séquences plasmidiques, n'a montré aucune hybridation croisée dans les conditions strictes avec les ADN des HPV5, 20, 23 et 36. En revanche, cette sonde a permis de mettre en évidence un fragment d'ADN de 1,9 Kb dans l'ADN extrait des lésions traitées par l'enzyme EcoRI, ainsi que des molécules d'ADN de HPV forme I (circulaires superenroulées) et forme II (circulaires ouvertes) dans l'ADN tumoral non traité. Une étude de la sensibilité de l'ADN de ce nouvel HPV à plusieurs enzymes de restriction a montré que l'enzyme SacI coupe une fois l'ADN viral. Après digestion de l'ADN tumoral par l'enzyme SacI, les molécules d'ADN de 8 Kb (taille du génome d'un HPV) ont été purifiées par centrifugation dans un gradient de saccharose, puis insérées par le site SacI dans l'ADN du plasmide SP65. Les plasmides recombinants ayant intégré l'ADN du nouvel HPV ont été sélectionnés par la méthode d'hybridation sur colonies, en utilisant une sonde d'ADN radioactive spécifique du fragment de 1,9 Kb dans des conditions strictes. Plusieurs Plasmides recombinants contenant la totalité des séquences virales ont été isolés. La coupure de l'ADN des plasmides recombinants par l'endonucléase SacI engendre un fragment de 8 Kb hybridant avec le fragment d'ADN de 1,9 Kb dans les conditions strictes. La coupure de l'ADN des plasmides recombinants et de la préparation d'ADN extrait des lésions, par le mélange des enzymes SacI et PvuII, génère les mêmes quatre fragments, dont le poids moélculaire total correspond à celui d'un génome de papillomavirus (8 Kb).

Une carte de restriction de l'ADN du nouvel HPV a été construite à partir de l'étude de la sensibilité de cet ADN à 16 enzymes de restriction. Vingt-huit sites de coupure ont été localisés. La carte ainsi établie est différente de celle des génomes des HPV identifiés à ce jour. L'homologie de séquence entre l'ADN du nouvel HPV et les ADN des HPV caractérisés à ce jour a été analysées par des expériences d'hybridation sur réplique effectuées dans des conditions strictes ou non strictes. Aucune homologie de séquence n'a été décelée dans des conditions strictes. En revanche, dans des conditions non strictes, une hybridation croisée significative, bien que faible, a été observée uniquement entre l'ADN du nouvel HPV et les ADN des HPV spécifiques de l'EV. Le virus isolé à partir de lésions de kératose actinique, observées chez un malade atteint d'EV, constitue donc un nouveau type d'HPV, provisoirement dénommé HPV50.

L'analyse comparative de la séquence nucléotidique du HPV8 et d'une petite région du génome du HPV50 a permis d'aligner la carte physique du HPV50 avec la carte génétique du HPV8 et de définir la position théorique des différents gènes portés par l'ADN du HPV50.

L'utilisation de sondes radioactives préparées à partir de l'ADN du HPV50 purifié a permis de déterminer le pouvoir pathogène de ce virus. Le HPV50 n'a pas été trouvé dans des verrues observées chez des sujets de la population générale (22 cas analysés), des patients immunosupprimés (27 cas) et des bouchers (26 cas), dans 18 cas de kératoacanthomes, dans 20 cas de maladie de Bowen cutanée, dans 25 cas de kératose actinique, dans 24 cas d'épithélioma basocellulaire et dans 22 cas d'épithélioma spinocelulaire. En revanche, l'ADN du HPV50 a été détecté dans l'ADN extrait de grattages des lésions bénignes de trois patients atteints d'EV sur les 31 étudiés.

Le HPV50 constitue donc un type additionnel d'HPV cutané associé à l'EV. Il convient de l'incorporer à tout mélange d'ADN de HPV destiné à la préparation de sondes moléculaires, en vue du diagnostic des types de HPV associés à l'EV.

Dans le tableau qui suit sont précisées les localisations putatives des principaux gènes et de la région intergénique du HPV50 sur la carte de ce génome :

| | Coordonnées des extrémités (%) | |
|---|---|---|
| | 5′ | 3′ |
| E6-E7 | 1,8 | 12,6 |
| E1 | 10,2 | 36 |
| E2 | 35 | 54,8 |
| L2 | 55,8 | 76,2 |
| L1 | 75,2 | 96,6 |
| Région intergénique | 96,6 | 1,8 |

### Clonage moléculaire et caractérisation d'un nouveau type d'HPV trouvé dans une tumeur de Buschkse Loewenstein (HPV54) :

Un nouveau type d'HPV a été mis en évidence dans l'ADN extrait d'une tumeur de Buschke Loewenstein du pénis, par hybridation, dans des conditions non strictes, avec une sonde radioactive spécifique du HPV18. Une étude de la sensibilité de l'ADN de cet HPV à plusieurs endonucléases de restriction a montré que l'endonucléase BamHI coupait une fois l'ADN viral. Après digestion de l'ADN extrait de la tumeur par l'endonucléase BamHI, la fraction renfermant des molécules d'ADN de 8 Kb (taille d'un génome d'HPV) a été purifiée par centrifugation dans un gradient de saccharose. Les molécules de 8 Kb ont été insérées par le site BamHI dans l'ADN du bactériophage lambda. Après encapsidation de l'ADN recombinant et transfection de bactéries hôtes (Escherichia coli, souche LA101), les bactériophages recombinants ayant inséré l'ADN du nouvel HPV ont été sélectionnés par la technique d'hybridation sur plages de lyse (Benton et Davis), en utilisant une sonde d'ADN radioactive spécifique du HPV18 dans des conditions non strictes. Plusieurs bactériophages recombinants contenant la totalité des séquences virales ont été isolés. La coupure de l'ADN des bactériophages recombinants et de la préparation d'ADN extrait de la tumeur, par le mélange des endonucléases BamHI et PstI, engendre les mêmes fragments, dont le poids moléculaire total correspond à celui d'un génome de HPV. L'ADN du nouvel HPV a été excisé de séquences d'ADN phagique et recloné dans le plasmide SP64.

Une carte de restriction de l'ADN du nouvel HPV SP64 a été construite à partir de l'étude de la sensibilité de cet ADN à 20 endonucléases de restriction. Trente-trois sites ont été localisés. Cette carte est différente de celle de tous les HPV isolés à ce jour. Le degré d'homologie entre l'ADN du nouvel HPV et l'ADN des types de HPV connus a été analysé par des expériences d'hybridation sur réplique, dans des conditions strictes ou non strictes, en utilisant une sonde d'ADN radioactive spécifique du nouvel HPV. Seule une homologie de séquence faible a été observée dans les conditions non strictes d'hybridation. Une hybridation croisée légèrement plus importante a été détectée entre l'ADN du nouvel HPV et les ADN des HPV associés aux lésions des membranes muqueuses. Le virus isolé à partir de la tumeur de Buschke Loewenstein constitue donc un nouveau type d'HPV, HPV54.

L'analyse, au microscope électronique, de molécules hétéroduplexes formées entre l'ADN du HPV54 et l'ADN du HPV16 ainsi que la détermination de la séquence nucléotidique d'un fragment d'ADN du HPV54 ont permis d'aligner la carte physique du HPV54 avec la carte génétique du HPV16 et de définir la position théorique des différents gènes portés par l'ADN du HPV54.

L'ADN du HPV54 a été recherché dans des préparations d'ADN obtenues de lésions génitales de 179 patients. HPV54 n'a pas été trouvé dans 67 spécimens de condylomes acuminés, 10 spécimens de tumeurs de Buschke Loewenstein, 14 spécimens de papulose bowenoïde, 17 spécimens de maladie de Bowen, 40 spéciments de carcinomes invasifs et 28 spcimens de néoplasies inraépithéliales du cervix,

Le HPV54 constitue un nouveau type de HPV très peu apparenté aux HPV génitaux caractérisés à ce jour. Il convient de l'incorporer à tout mélange destiné à la préparation de sondes moléculaires, en vue du diagnostic des types de HPV associés aux lésions génitales.

Dans le tableau qui suit sont précisées les localisations putatives des principaux gènes et de la région intergénique du HPV54 sur la carte de ce génome :

| | Coordonnées des extrémités (%) | |
|---|---|---|
| | 5′ | 3′ |
| E6-E7 | 1,8 | 10,8 |
| E1 | 10,8 | 35,6 |
| E2 | 34,5 | 48,7 |
| L2 | 52,3 | 71,5 |
| L1 | 69,9 | 90,5 |
| Région intergénique | 90,5 | 1,8 |

### Clonage moléculaire et caractérisation d'un nouveau type d'HPV, associé à des condylomes acuminés (HPV55) :

Un nouveau type d'HPV a été mis en évidence dans l'ADN extrait d'un condylome acuminé du pénis, par hybridation dans des conditions non strictes, avec une sonde d'ADN radioactive spécifique du HPV6. Une étude de la sensibilité à plusieurs endonucléases de restriction a montré que l'ADN de cet HPV est coupé deux fois par l'endonucléase EcoRI engendrant deux fragments d'ADN de poids moléculaire 5,65 et 2,15 Kilobases (Kb), dont la somme (8 Kb) correspond à la taille d'un génome de HPV. Après digestion de l'ADN tumoral par l'enzyme EcoRI, les molécules d'ADN de taille inférieure à 7 Kb ont été insérées par le site EcoRI dans l'ADN du bactériophage lambda gt10. Après encapsidation de l'ADN recombinant et infection de bactéries Escherichia Coli K12 (souche C600), les bactériophages recombinants contenant les fragments subgénomiques du nouvel HPV ont été sélectionnés par la technique d'hybridation sur plages de lyse (Benton et Davis), en utilisant une sonde d'ADN du HPV6, dans des conditions non strictes d'hybridation. Plusieurs bactériophages recombinants contenant l'un ou l'autre des deux fragments correspondant au génome du nouvel HPV ont été caractérisés. Le traitement de l'ADN tumoral extrait des lésions et des bactériophages recombinants par l'endonucléase EcoRI engendrait les deux fragments attendus de 5,65 et 2,15 Kb. Les deux fragments ont été excisés des séquences d'ADN phagique et reclonés dans le plasmide pGem4. Une carte de restriction de l'ADN du nouvel HPV a été construite à partir de l'étude de la sensibilité de cet ADN à 15 endonucléases de restriction. Dix-neuf sites de coupure ont été localisés. Cette carte est différente de celle de tous les HPV caractérisés à ce jour. L'homologie de séquence entre l'ADN du nouvel HPV et l'ADN des HPV connus a été analysée par des expériences d'hybridation sur réplique effectuées dans des conditions strictes, en utilisant une sonde d'ADN radioactif spécifique du nouvel HPV. Une hybridation partielle a été observée avec l'ADN du nouvel HPV et l'ADN du HPV13 et une hybridation croisée plus faible a été décelée avec les ADN des HPV6 et 11. Le degré d'homologie d'ADN a été évalué par des expériences d'hybridation en milieu liquide à saturation, suivies par une digestion des hybrides par la nucléase S1 ; il a été estimé respectivement à 20,10 et 10 % entre l'ADN du nouvel HPV et les ADN des HPV13, 6 et 11. Le virus isolé d'un condylome du pénis constitue donc un nouveau type d'HPV, HPV55.

L'analyse, au microscope électronique, de molécules hétéroduplexes formées entre l'ADN du HPV55 et l'ADN du HPV11 et l'établissement de la séquence nucléotidique d'un fragment d'ADN du HPV55 a permis d'aligner la carte physique de l'ADN du HPV55 avec la carte génétique de l'HPV11 et de définir la position théorique des différents gènes portés par l'ADN du HPV55.

L'utilisation de sondes radioactives préparées à partir de l'ADN du HPV55 purifié a permis d'étudier le pouvoir pathogène de ce virus. L'ADN du HPV55 n'a été mis en évidence que dans un condylome anal d'une autre patiente sur les 67 cas étudiés. Le HPV55 n'a pas été détecté dans l'ADN extrait d'autres types de lésions génitales prélevées chez 96 patients additionnels.

Le HPV55, bien que rare, doit être incorporé à tout mélange de HPV destiné à la préparation de sondes, en vue du diagnostic de types de HPV associés aux lésions génitales.

Dans le tableau qui suit sont précisées les localisations putatives des principaux gènes et de la région intergénique du HPV55 sur la carte de ce génome :

| | Coordonnées des extrémités (%) | |
|---|---|---|
| | 5′ | 3′ |
| E6-E7 | 1,3 | 10,4 |
| E1 | 9 | 35 |
| E2 | 34 | 48,2 |
| L2 | 55,6 | 72,9 |
| L1 | 71,4 | 91,7 |
| Région intergénique | 91,7 | 1,3 |

D'une façon générale l'invention concerne donc également tout ADN recombinant contenant l'ADN-HPV susdit ou des fragments de cet ADN-HPV, en particulier des sondes d'hybridation formées de ces ADNs recombinants et spécialement adaptées à la détection d'une infection par le papillomavirus selon l'invention ou d'un variant de ce papillomavirus. Ces sondes peuvent, soit être marquées elles-mêmes, soit être modifiées au niveau de certains nucléotides, notamment en vue de leur couplage, direct ou indirect, avec un marqueur distinct. Il va de soi que dans ces sondes les parties étrangères à la séquence nucléotidique correspondante à l'ADN du papillomavirus et provenant normalement d'un vecteur de clonage, sont telles qu'elles ne risquent pas d'hybrider dans des conditions stringentes avec les autres acides nucléiques éventuellement contenus dans l'échantillon testé pour sa teneur éventuelle en ADN du papillomavirus correspondant ou de l'un de ses variants.

Le procédé selon l'invention pour le diagnostic in vitro sur un échantillon biologique à tester d'une infection par un papillomavirus (HPV49, HPV50, HPV54, HPV55) ou par un variant, est donc caractérisé par la mise en contact d'une sonde telle que ci-dessus définie avec les acides nucléiques de cet échantillon, le cas échéant préalablement rendus accessibles à la sonde, de préférence dans des conditions d'hybridation stringentes et par la détection de l'hybride formé entre l'ADN viral recherché éventuellement présent dans l'échantillon et ladite sonde.

Le procédé selon l'invention est particulièrement avantageux pour la détection in vitro du
- HPV49 : dans des verrues cutanées (en particulier, verrues vulgaires et plantaires) et le diagnostic différentiel de l'épidermodysplasie verruciforme,
- HPV50 : dans l'épidermodysplasie verruciforme, les néoplasies intra-épithéliales et les cancers cutanés,
- HPV54 : dans des néoplasies génitales et les cancers du col de l'utérus,
- HPV55 : dans des néoplasies génitales et cancers du col de l'utérus, les condylomes et papillomes.

Dans une variante de l'invention, la sonde est associée à des sondes dérivées d'autres papillomavirus, en particulier de ceux désignés ci-après :
en ce qui concerne HPV49, en association avec les HPV1, 2d, 4 ou, en variante, en association avec les HPV3, 10a, 10b, 28, 29,
en ce qui concerne HPV50, en association avec les HPV5, 17a, 24, ou en variante encore, en association avec les HPV5, 8, 14b, 36,
en ce qui concerne HPV54, en association avec les HPV16, 18, 33, 39, ou en variante encore, en association avec les HPV6, 11, 42,
en ce qui concerne HPV55, en association avec les HPV6, 11, 42.

Chacune des sondes selon l'invention ou les mélanges contenant la susdite sonde peuvent notamment être utilisés comme suit, étant naturellement entendu que les essais de diagnostic décrits ne sauraient être considérés comme limitatifs des conditions d'emploi des sondes ou mélanges de sondes selon l'invention.

Dans l'exemple considéré, il s'agit par exemple d'identifier l'HPV dans une biopsie, dans des cellules obtenues par grattage de lésions, ou dans des coupes de biopsies fixées par le mélange de Carnoy (éthanol, chloroforme, acide acétique 6:3:1) et incluses dans la paraffine. L'examen nécessite l'extraction préalable de l'ADN des prélèvements selon des méthodes dont le principe est connu et met en jeu l'analyse de cet ADN par des expériences d'hybridation moléculaire, effectuées dans des conditions strictes ou moins strictes, à l'aide de sondes radioactives (marquées au ³²p ou au ³⁵S) préparées à partir de l'HPV selon l'invention ou de mélanges d'ADNs ou d'HPVs.

Plusieurs méthodes d'hybridation peuvent être utilisées. On peut, par exemple, mettre en oeuvre la méthode d'hybridation sur tache. Cette méthode comporte, après dénaturation de l'ADN, le dépôt d'une quantité aliquote d'ADN sur des membranes (nitrocellulose ou "Genescreenplus"), l'hybridation de chaque membrane, dans les conditions usuelles, avec un mélange de sondes et la detection des hybrides radioactifs par exposition des membranes au contact d'un film radiographique. On peut aussi utiliser une méthode d'hybridation sur réplique. Cette méthode comprend la séparation électrophorétique en gel d'agarose des fragments d'ADN engendrés après traitement de l'ADN par des enzymes de restriction, le transfert des fragments, après dénaturation alcaline, sur des membranes (nitrocellulose, "Genescreenplus") et leur hybridation, dans les conditions usuelles, avec différents mélanges de sondes. La formation d'hybrides radioactifs est détectée après exposition des membranes au contact d'un film radiographique.

Les sondes radioactives sont constituées soit par des ADNs d'HPVs marqués par la méthode de "nick-translation", soit par des ARNs préparés par transcription d'ADNs viraux insérés dans un vecteur, par exemple de type SP6. L'utilisation de sondes radioactives présente l'avantage d'une grande sensibilité, mais ceci n'exclut pas l'utilisation de sondes non radioactives par exemple biotinylées et susceptibles d'être reconnues par des anticorps soit marqués eux-mêmes, soit eux-mêmes reconnus par des anticorps portant un marqueur enzymatique, fluorescent, etc...

L'invention concerne également des cultures cellulaires compétentes transformées avec des ADNs recombinants du type sus-indiqué, en particulier ceux dans lesquels la séquence nucléotidique correspondant à l'ADN du papillomavirus est placée sous le contrôle d'éléments de transcription et de régulation de cette séquence nucléotidique dans ladite culture cellulaire.

Par conséquent, elle concerne également les produits d'expression de ces ADNs recombinants et les anticorps correspondants susceptibles d'être produits contre ces produits d'expression.

A ce titre, l'invention concerne les polypeptides résultant notamment de l'expression des gènes E1, E2, E6, E7, L1, L2, qui peuvent ainsi être produits, correspondent respetivement à chacun des papillomavirus.

Le procédé selon l'invention pour la production de ces polypeptides comprend par conséquent la transformation de cultures cellulaires compétentes avec l'un des ADNs recombinants tels que ci-dessus définis, de façon telle que la séquence nucléotidique correspondant à l'une desdites protéines puisse être exprimée dans cet hôte cellulaire, la récupération de ces polypeptides à partir des produits synthétisés par l'hôte cellulaire compétent et la purification (par exemple par mise en contact des produits d'expression préalablement extraits des cultures cellulaires ou du milieu dans lequel celles-ci ont été développées, avec des anticorps préalablement formés contre de tels polypeptides).

Les produits d'expression des séquences L2 de chacun des génomes des papillomavirus selon l'invention présentent cependant un intérêt tout particulier, en ce qu'ils peuvent eux-mêmes être utilisés pour la production in vivo d'anticorps susceptibles de reconnaître les produits d'expression du gène L2 dans des prélèvements biologiques affectés par le papillomavirus correspondant et pas par un papillomavirus de type différent, et plus particulièrement lorsque les préparations du genre en question ont préalablement été fixées.

L'invention concerne encore des polypeptides hybrides contenant les polypeptides susdits et dérivés respectivement des papillomavirus correspondants, par exemple la protéine L2 fusionnée à d'autres séquences polypeptidiques, dans la mesure où celles-ci ne modifient pas de façon essentielle les propriétés immunogènes de la protéine L2. La présence de ces autres fragments polypeptidiques peut notamment résulter du mode de production utilisé de ces polypeptides hybrides, par des procédés de génie génétique. Par exemple, ces polypeptides hybrides contiennent une séquence dérivée de la bêta-galactosidase. De tels produits peuvent notamment être obtenus par transformation de E. coli avec des vecteurs appropriés (phages ou plasmides) modifiés par tout ou partie de l'opéron lactose et comportant en outre, insérée en aval du promoteur de l'opéron-lactose (ou de tout autre promoteur approprié, par exemple de phage lambda), la séquence nucléotidique dérivée d'un gène L2 issu du papillomavirus concerné selon l'invention. On a avantageusement recours à des plasmides ou phages de ce type comprenant une partie au moins du gène de la bêta-galactosidase de l'opéron-lactose.

Les polypeptides selon l'invention peuvent également, lorsqu'ils ont été purifiés, être mis en oeuvre dans des techniques de purification des anticorps qui leur correspondent, notamment à partir de sérums d'animaux qui avaient été immunisés par ces polypeptides. En particulier, ces polypeptides peuvent être fixés sur des colonnes d'affinité. Les opérations de purification des anticorps consistent alors à faire passer le sérum les contenant au contact de colonnes d'affinité portant les susdits polypeptides. Les anticorps sélectivement fixés sur ces colonnes peuvent ensuite être récupérés par dissociation des complexes antigènes-anticorps, au moyen d'un tampon approprié, présentant une force ionique adéquate, par exemple une solution d'un sel tel que l'acétate d'ammonium. On peut également avoir recours à des solutions acidifiées.

L'invention concerne également un procédé de production d anticorps contre lesdits polypeptides, en particulier contre les produits d'expression des gènes E6, E7 ou, de préférence L2 de chacun des papillomavirus selon l'invention, en particulier contre les produits d'expression des gènes E6, E7 ou, de préférence L2, du papillomavirus selon l'invention, ce procédé comprenant l'immunisation d'un hôte vivant approprié avec lesdits polypeptides et la récupération des anticorps formés à partir d'un sérum de l'hôte immunisé, notamment par mise en contact de ces sérums avec les polypeptides correspondant à l'état purifié et par la récupération de ces anticorps à partir des complexes antigène-anticorps formés.

L'invention concerne enfin les compositions mettant en jeu les anticorps selon l'invention (ou groupes contenant ces anticorps en association avec des anticorps issus d'autres papillomavirus), en particulier les compositions contenant les anticorps dérivés des compositions ou "cocktails" d'ADN-HPVs répertoriés plus haut et sur lesquels il est encore revenu dans ce qui suit (ou groupes d'anticorps correspondants).

En particulier, l'invention concerne les anticorps ou mélanges d'anticorps correspondants, réputés être souvent présents dans un type d'affection donné. Ces anticorps préalablement purifiés en association avec un véhicule pharmaceutique approprié sont alors susceptibles d'être mis en oeuvre par administration, notamment parentérale, au malade concerné, dans le traitement de l'affection donnée, dès lors que celle-ci aura été diagnostiquée cliniquement, à l'issue d'un essai de diagnostic in vitro sur un prélèvement histologique ou cytologique provenant de ce malade, ou qu'un essai de diagnostic in vivo aura révélé l'appartenance du papillomavirus infectieux à un type semblable à celui de l'un des papillomavirus de l'ensemble sus-indiqué. Ces sérums sont alors susceptibles de provoquer une régression des infections induites par le papillomavirus selon l'invention.

Ces anticorps peuvent plus particulièrement être mis en oeuvre dans des essais de diagnostic d'une infection à l'égard de l'un des papillomavirus selon l'invention ou de variants de ces papillomavirus, dans la mesure où des coupes histologiques provenant des personnes infectées peuvent également contenir des produits d'expression de certains des gènes de structure, notamment L2, des mêmes papillomavirus ou variants correspondants.

Avantageusement aussi ces anticorps, éventuellement associés à des anticorps provenant d'autres papillomavirus dans les conditions sus-indiquées peuvent être utilisés pour le diagnostic in vitro, en particulier :
- HPV49 : dans des verrues cutanées (en particulier, verrues vulgaires et plantaires) et le diagnostic différentiel de l'épidermodysplasie verruciforme,
- HPV50 : dans l'épidermodysplasie verruciforme, les néoplasies intra-épithéliales et les cancers cutanés,
- HPV54 : dans des néoplasies génitales et les cancers du col de l'utérus,
- HPV55 : dans des néoplasies génitales et cancers du col de l'utérus, les condylomes et papillomes.

L'invention concerne donc encore un procédé de diagnostic in vitro comprenant la mise en contact de coupes histopathologiques provenant de lésions induites chez les personnes concernées par l'un des papillomavirus avec l'anticorps ou les anticorps choisis dans des conditions permettant la production d'un complexe antigène-anticorps et la détection de ce complexe antigène-anticorps. Avantageusement, la détection se fait sur des préparations fixées au préalable dans des conditions dissociantes, par exemple avec le milieu ou mélange de CARNOY déjà mentionné plus haut (également décrit dans l'ouvrage de L. LISON, intitulé "Histochimie et cytochimie aniumales".

Les anticorps anti-L2 éventuellement fixés peuvent être reconnus par d'autres anticorps formés contre les premiers, ces autres anticorps portant des marqueurs appropriés, de préférence non radioactifs. Ces marqueurs sont par exemple de nature enzymatique ou fluorescente.

Les anticorps ainsi sélectionnés, comme les sondes d'hybridation sus-définies qui leur correspondent, peuvent par conséquent être utilisées pour diagnostiquer in vitro les types d'affections correspondant.

L'invention concerne enfin les compositions vaccinantes correspondantes contenant une ou de préférence plusieurs protéines L2, en association avec un véhicule pharmaceutiquement acceptable adapté au mode d'administration choisi, notamment par voie parentérale utilisable pour protéger les personnes soumises à de hauts risques d'être atteints par une affection due au papillomvirus correspondant.

Les souches des ADN-HPV susdits ont été déposés à la CNCM le 6 mai 1988 sous les numéros de dépôt :

| | |
|---|---|
| - PGEM 4 HPV 49 | I-754 |
| - PSP 65 HPV 50 | I-755 |
| - PSP 64 HPV 54 | I-756 |
| - PGEM 4 HPV 55 A | I-757 |
| - PGEM 4 HPV 55 B | I-758. |

L'invention concerne aussi les fragments plus courts, dérivés des ADNs susdits, par exemple de 15-25 nucléotides. Ces fragments plus courts sont utilisables pour la constitution d'amorces pour la mise en oeuvre d'une variante mettant en oeuvre la technique dite PCR (réaction de chaîne avec polymérase, de l'anglais "Polymerase Chain Reaction") telle que décrite dans les brevets américains n° 4.683.202 et 4.683.195 et la demande de brevet européen n° 200.362.

## Revendications

1. ADN de papillomavirus HPV49 déposé à la CNCM sous le numéro de dépôt I-754, caractérisé par la carte de restriction de la figure 1, et de tout variant de ce papillomavrius appartenant au même sous-type et capable de s'hybrider avec ledit papillomavirus dans des conditions stringentes.

2. ADN de papillomavirus HPV50 déposé à la CNCM sous le numéro de dépôt I-755, caractérisé par la carte de restriction de la figure 2, et de tout variant de ce papillomavirus appartenant au même sous-type et capable de s'nybrider avec ledit papillomavirus dans des conditions stringentes.

3. ADN de papillomavirus HPV54 déposé à la CNCM sous le numéro de dépôt I-756, caractérisé par la carte de restriction de la figure 3, et de tout variant de ce papillomavirus appartenant au même sous-type et capable de s'hybrider avec ledit papillomavirus dans des conditions stringentes.

4. ADN de papillomavirus HPV55, notamment HPV55A déposé à la CNCM sous le numéro de dépôt I-757 ou HPV55B déposé à la CNCM sous le numéro de dépôt I-758 ainsi que de tous les variants de ces papillomavirus appartenant au même sous-type et capables de s'hybrider avec lesdits papillomavirus dans des conditions stringentes.

5. Sonde d'hybridation constituée par un ADN dérivé ou constitué par un fragment de l'un des ADNs selon l'une quelconque des revendications 1 à 4, ladite sonde étant capable de s'hybrider avec l'un des ADNs selon l'une quelconque des revendications 1 à 4, notamment dans des conditions strictes, de façon à pouvoir détecter l'hybride formé.

6. Polypeptide correspondant au produit d'expression de l'un des gènes de structure de l'un des ADNs de papillomavirus, selon l'une quelconque des revendications 1 à 4.

7. Polypeptide selon la revendication 6, caractérisé en ce qu'il consiste plus particulièrement en un produit d'expression de la séquence L2 du génome du papillomavirus concerné.

8. Anticorps dirigés contre l'un des polypeptides selon la revendication 6 ou la revendication 7.

9. Procédé de détection in vitro sur un échantillon biologique à tester d'une infection par un papillomavirus du type HPV49 déposé à la CNCM sous le numéro de dépôt I-754, HPV50 déposé à la CNCM sous le numéro de dépôt I-755, HPV54 déposé à la CNCM sous le numéro de dépôt I-756 ou HPV55 notamment HPV55A déposé à la CNCM sous le numéro de dépôt I-757 ou HPV55B déposé à la CNCM sous le numéro de dépôt I-758, caractérisé par la mise en contact d'une sonde correspondante, telle que définie dans la revendication 5, avec les acides nucléiques de cet échantillon, le cas échéant préalablement rendus accessibles à la sonde, de préférence dans des conditions d'hybridation stringente, et par la détection de l'hybride formé entre l'ADN viral recherché, éventuellement présent dans l'échantillon et ladite sonde.

10. Procédé selon la revendication 9, caractérisé en ce que la sonde est dérivée de HPV49 et que le diagnostic in vitro est orienté vers la détection de verrues cutanées (en particulier, verrues vulgaires et plantaires) et le diagnostic différentiel de l'épidermodysplasie verruciforme.

11. Procédé selon la revendication 9, caractérisé en ce que la sonde est dérivée de HPV50 et en ce que le diagnostic in vitro est orienté vers la détection d'épidermodysplasies verruciformes, de néoplasies intraépithéliales et de cancers cutanés.

12. Procédé selon la revendication 9, caractérisé en ce que la sonde est dérivée de HPV54 et en ce que le diagnostic in vitro est orienté vers la détection de néoplasies génitales et de cancers du col de l'utérus.

13. Procédé selon la revendication 9, caractérisé en ce que la sonde est dérivée de HPV55 et en ce que le diagnostic in vitro est orienté vers la détection de néoplasies génitales et cancers du col de l'utérus, de condylomes et papillomes.

14. Application des anticorps selon la revendication 8 au diagnostic in vitro d'une infection par un papillomavirus du type HPV49 déposé à la CNCM sous le numéro de dépôt I-754, HPV50 déposé à la CNCM sous le numéro de dépôt I-755, HPV54 déposé à la CNCM sous le numéro de dépôt I-756 ou HPV55 notamment HPV55A déposé à la CNCM sous le numéro de dépôt I-757 ou HPV55B déposé à la CNCM sous le numéro de dépôt I-758, caractérisée par la mise en contact d'un anticorps, tel que défini dans la revendication 8, avec un échantillon biologique provenant de la personne commise au diagnostic in vitro et par la détection du complexe antigène-anticorps formé, cette détection permettant le diagnostic in vitro, selon les anticorps mis en oeuvre :
- pour ceux dérivés de HPV49 : des verrues cutanées (en particulier verrues vulgaires et plantaires) et du diagnostic différentiel de l'épidermodysplasie verruciforme,
- pour ceux dérivés de HPV50 : des épidermodysplasies verruciformes, les néoplasies intra-épithéliales et les cancers cutanés,
- pour ceux dérivés de HPV54, de néoplasies génitales et les cancers du col de l'utérus,
- pour ceux dérivés de HPV55 : des néoplasies génitales et des cancers du col de l'utérus, des condylomes et papillomes.

15. Application selon la revendication 14, caractérisée en ce que l'anticorps mis en oeuvre est dirigé contre un produit d'expression de la séquence L2 du génome du papillomavirus concerné.

## Claims

1. DNA of papillomavirus HPV49 deposited at CNCM under deposit number I-754, characterized by the restriction map of Figure 1, and of any variant of this papillomavirus belonging to the same subtype and capable of hybridizing with the said papillomavirus under stringent conditions.

2. DNA of papillomavirus HPV50 deposited at CNCM under deposit number I-755, characterized by the restriction map of Figure 2, and of any variant of this papillomavirus belonging to the same subtype and capable of hybridizing with the said papillomavirus under stringent conditions.

3. DNA of papillomavirus HPV54 deposited at CNCM under deposit number I-756, characterized by the restriction map of Figure 3, and of any variant of this papillomavirus belonging to the same subtype and capable of hybridizing with the said papillomavirus under stringent conditions.

4. DNA of papillomavirus HPV55, especially HPV55A deposited at CNCM under deposit number I-757 or HPV55B deposited at CNCM under deposit number I-758 as well as of all the variants of these papillomaviruses belonging to the same subtype and capable of hybridizing with the said papillomaviruses under stringent conditions.

5. Hybridization probe consisting of a DNA derived from or consisting of a fragment of one of the DNAs according to any one of Claims 1 to 4, the said probe being capable of hybridizing with one of the DNAs according to any one of Claims 1 to 4, especially under strict conditions, so as to be able to detect the hybrid formed.

6. Polypeptide corresponding to the product of expression of one of the structural genes of one of the papillomavirus DNAs, according to any one of Claims 1 to 4.

7. Polypeptide according to Claim 6, characterized in that it consists more particularly of a product of expression of sequence L2 of the genome of the relevant papillomavirus.

8. Antibodies directed against one of the polypeptides according to Claim 6 or Claim 7.

9. Process for the detection in vitro, on a biological sample to be tested, of an infection by a papillomavirus of the type HPV49 deposited at CNCM under deposit number I-754, HPV50 deposited at CNCM under deposit number I-755, HPV54 deposited at CNCM under deposit number I-756 or HPV55, especially HPV55A deposited at CNCM under deposit number I-757 or HPV55B deposited at CNCM under deposit number I-758, characterized by the bringing into contact of a corresponding probe, as defined in Claim 5, with the nucleic acids of this sample, which have where appropriate previously been made accessible to the probe, preferably under stringent hybridization conditions, and by the detection of the hybrid formed between the desired viral DNA which may be present in the sample and the said probe.

10. Process according to Claim 9, characterized in that the probe is derived from HPV49 and that the in vitro diagnosis is oriented towards the detection of skin verruca (in particular, verruca vulgaris and verruca plantaris) and the differential diagnosis of epidermodysplasia verruciformis.

11. Process according to Claim 9, characterized in that the probe is derived from HPV50 and in that the in vitro diagnosis is oriented towards the detection of epidermodysplasia verruciformis, of intraepithelial neoplasias and of skin cancers.

12. Process according to Claim 9, characterized in that the probe is derived from HPV54 and in that the in vitro diagnosis is oriented towards the detection of genital neoplasias and of cervical cancers.

13. Process according to Claim 9, characterized in that the probe is derived from HPV55 and in that the in vitro diagnosis is oriented towards the detection of genital neoplasias and of cervical cancers, of condylomas and papillomas.

14. Application of the antibodies according to Claim 8 to the in vitro diagnosis of an infection by a papillomavirus of the type HPV49 deposited at CNCM under deposit number I-754, HPV50 deposited at CNCM under deposit number I-755, HPV54 deposited at CNCM under deposit number I-756 or HPV55, especially HPV55A, deposited at CNCM under deposit number I-757 or HPV55B deposited at CNCM under deposit number I-758, characterized by the bringing into contact of an antibody, as defined in Claim 8, with a biological sample obtained from the person designated for the in vitro diagnosis and by the detection of the antigen-antibody complex formed, this detection permitting the in vitro diagnosis, according to the antibodies used:
- for those derived from HPV49: skin verruca (in particular verruca vulgaris and verruca plantaris) and differential diagnosis of epidermodysplasia verruciformis,
- for those derived from HPV50: epidermodysplasia verruciformis, intraepithelial neoplasias and skin cancers,
- for those derived from HPV54, genital neoplasias and cervical cancers,
- for those derived from HPV55: genital neoplasias and cervical cancers, condylomas and papillomas.

15. Application according to Claim 14, characterized in that the antibody used is directed against a product of expression of the sequence L2 of the genome of the relevant papillomavirus.

## Patentansprüche

1. DNA des Papillomavirus HPV49, hinterlegt bei CNCM unter der Hinterlegungsnummer I-754, gekennzeichnet durch die Restriktionskarte gemäß Figur 1, und jeder Variante des Papillomavirus, die zum gleichen Untertyp gehört und mit dem Papillomavirus unter stringenten Bedingungen hybridisieren kann.

2. DNA des Papillomavirus HPV50, hinterlegt bei CNCM unter der Hinterlegungsnummer I-755, gekennzeichnet durch die Restriktionskarte gemäß Figur 2, und jeder Variante des Papillomavirus, die zu dem gleichen Untertyp gehört und mit dem Papillomavirus unter stringenten Bedingungen hybridisieren kann.

3. DNA des Papillomavirus HPV54, hinterlegt bei CNCM unter der Hinterlegungsnummer I-756, gekennzeichnet durch die Restriktionskarte gemäß Figur 3, und jeder Variante des Papillomavirus, die zum gleichen Untertyp gehört und mit dem Papillomavirus unter stringenten Bedingungen hybridisieren kann.

4. DNA des Papillomavirus HPV55, insbesondere HPV55A, hinterlegt bei CNCM unter der Hinterlegungsnummer I-757, oder HPV55B, hinterlegt bei CNCM unter der Hinterlegungsnummer I-758, sowie aller Varianten der Papillomaviren, die zum gleichen Untertyp gehören und mit den Papillomaviren unter stringenten Bedingungen hybridisieren können.

5. Hybridisierungssonde, bestehend aus einer DNA, die von einem Fragment einer der DNAs nach einem der Ansprüche 1 bis 4 stammt oder daraus besteht, wobei die Sonde mit den DNAs nach einem der Ansprüche 1 bis 4 hybridisieren kann, insbesondere unter stringenten Bedingungen, so daß das gebildete Hybrid nachgewiesen werden kann.

6. Polypeptid, entsprechend einem Expressionsprodukt eines der Strukturgene einer der Papillomavirus-DNAs nach einem der Ansprüche 1 bis 4.

7. Polypeptid nach Anspruch 6, dadurch gekennzeichnet, daß es insbesondere aus einem Expressionsprodukt der Sequenz L2 des betreffenden Papillomavirusgenoms besteht.

8. Antikörper gegen eines der Polypeptide nach Anspruch 6 oder 7.

9. Verfahren zum in vitro-Nachweis in einer biologischen Probe einer Infektion durch ein Papillomavirus vom Typ HPV49, hinterlegt bei CNCM unter der Hinterlegungsnummer I-754, HPV50, hinterlegt bei CNCM unter der Hinterlegungsnummer I-755, HPV54, hinterlegt bei CNCM unter der Hinterlegungsnummer I-756, oder HPV55, insbesondere HPV55A, hinterlegt bei CNCM unter der Hinterlegungsnummer I-757, oder HPV55B, hinterlegt bei CNCM unter der Hinterlegungsnummer I-758, gekennzeichnet durch das Inkontaktbringen einer entsprechenden Sonde gemäß der Definition in Anspruch 5 mit den Nucleinsäuren der Probe, wobei diese gegebenenfalls vorher für die Sonde zugänglich gemacht wurden, vorzugsweise unter stringenten Hybridisierungsbedingungen, und durch den Nachweis des zwischen der untersuchten viralen DNA, die gegebenenfalls in der Probe vorliegt, und der Sonde gebildeten Hybrids.

10. Verfahren nach Anspruch 9, dadurch gekennzeichnet, daß die Sonde von HPV49 stammt und daß die in vitro-Diagnose auf den Nachweis von Hautwarzen gerichtet ist (insbesondere gemeine Warzen und plantare Warzen) und die Differentialdiagnose der Epidermodysplasia verruciformis.

11. Verfahren nach Anspruch 9, dadurch gekennzeichnet, daß die Sonde von HPV50 stammt und daß die in vitro-Diagnose auf den Nachweis von Epidermodysplasia verruciformis, intraepithelialen Neoplasien und Hautkrebs gerichtet ist.

12. Verfahren nach Anspruch 9, dadurch gekennzeichnet, daß die Sonde von HPV54 stammt und daß die in vitro-Diagnose auf den Nachweis von Genitalneoplasien und Krebs des Gebärmutterhalses gerichtet ist.

13. Verfahren nach Anspruch 9, dadurch gekennzeichnet, daß die Sonde von HPV55 stammt und daß die in vitro-Diagnose auf den Nachweis von Genitalneoplasien und Krebs des Gebärmutterhalses, von Condylomen und Papillomen gerichtet ist.

14. Verwendung der Antikörper nach Anspruch 8 für die in vitro-Diagnose einer Infektion durch ein Papillomavirus vom Typ HPV49, hinterlegt bei CNCM unter der Hinterlegungsnummer I-754, HPV50, hinterlegt bei CNCM unter der Hinterlegungsnummer I-755, HPV54, hinterlegt bei CNCM unter der Hinterlegungsnummer I-756, oder HPV55, insbesondere HPV55a, hinterlegt bei CNCM unter der Hinterlegungsnummer I-757, oder HPV55B, hinterlegt bei CNCM unter der Hinterlegungsnummer I-758, gekennzeichnet durch das Inkontaktbringen eines Antikörpers gemäß der Definition in Anspruch 8 mit einer biologischen Probe, die von der für die in vitro-Diagnose vorgesehenen Person stammt, und durch den Nachweis des gebildeten Antigen-Antikörper-Komplexes, wobei der Nachweis die folgende in vitro-Diagnose je nach den verwendeten Antikörpern erlaubt:
- für die Derivate von HPV49: Hautwarzen (insbesondere gemeine und plantare Warzen) und die Differentialdiagnose der Epidermodysplasia verruciformis,
- für die Derivate von HPV50: Epidermodysplasia verruciformis, intra-epitheliale Neoplasien und Hautkrebs,
- für die Derivate von HPV54: Genitalneoplasien und Krebs des Gebärmutterhalses,
- für die Derivate von HPV55: Genitalneoplasien und Krebs des Gebärmutterhalses, Condylomen und Papillomen.

15. Verwendung nach Anspruch 14, dadurch gekennzeichnet, daß der verwendete Antikörper gegen ein Expressionsprodukt der Sequenz L2 des betreffenden Papillomavirusgenoms gerichtet ist.
